(19) **Europäisches Patentamt**

**European Patent Office**

**Office européen des brevets**

(11) **EP 1 382 387 A1**

(12) **EUROPEAN PATENT APPLICATION**
published in accordance with Art. 158(3) EPC

(43) Date of publication:
**21.01.2004 Bulletin 2004/04**

(51) Int Cl.[7]: **B01J 23/89**, C07C 45/29,
C07C 49/403

(21) Application number: **02708740.2**

(22) Date of filing: **29.03.2002**

(86) International application number:
**PCT/JP2002/003223**

(87) International publication number:
**WO 2002/078841 (10.10.2002 Gazette 2002/41)**

(84) Designated Contracting States:
**AT BE CH CY DE DK ES FI FR GB GR IE IT LI LU
MC NL PT SE TR**

(30) Priority: **29.03.2001 JP 2001097307**

(71) Applicant: **Sued-Chemie Catalysts Japan, Inc.
Shibuya-ku, Tokyo 151-0053 (JP)**

(72) Inventors:
• **KITAMURA, Tadakuni
c/o SUED-CHEMIE CAT.JAPAN, INC.
Shibuya-ku, Tokyo 151-0053 (JP)**

• **SUGETA, Moriyasu
c/o SUED-CHEMIE CAT. JAPAN, INC.
Nei-gun, Toyama 939-2753 (JP)**
• **OKUHARA, Kazuharu
c/o SUED-CHEMIE CAT. JAPAN, INC.
Nei-gun, Toyama 939-2753 (JP)**

(74) Representative: **Westendorp, Michael, Dr.
Patentanwälte
Splanemann Reitzner
Baronetzky Westendorp
Rumfordstrasse 7
80469 München (DE)**

(54) **CATALYST FOR DEHYDROGENATION OF CYCLOHEXANOL AND METHOD FOR PREPARATION THEREOF**

(57) A copper oxide based catalyst for dehydrogenation of cyclohexanol, which is a copper oxide-zinc oxide based catalyst or a copper oxide-silicon oxide based catalyst further comprising one of palladium, platinum and ruthenium in a very small amount; and a method for preparing the copper oxide based catalyst which comprises combining the co-precipitation method or the kneading method and the spray method. The copper oxide based catalyst for dehydrogenation of cyclohexanol exhibits high activity and high selectivity, and thus may be used for producing cyclohexanone at a reduced reaction temperature and/or with an enhanced yield, as compared to a conventional catalyst.

## Description

### Technical Field:

This invention relates to a catalyst for preparing cyclohexanone through dehydrogenation of cyclohexanol at a low temperature of 220 °C to 260 °C and a method for preparing the catalyst.

### Background of the Art

Usually, cyclohexanone is industrially produced by a method of dehydrogenating cyclohexanol, including a method of dehydrogenation at a low temperature of 220 °C to 260 °C and a method of dehydrogenation at a high temperature of 350 °C to 450 °C. For dehydrogenation of cyclohexanol at the low temperature, usually a copper oxide based catalyst is used. The previously known catalyst mainly composes copper oxide, to which metals such as Zn, Cr, Fe, Ni, alkali metals, alkaline earth metals, and thermally stable metal oxides such as Al, Si, Ti are added.

Further, the copper oxide based catalysts added with noble metals can be disclosed in less number of patents. For example, Pd-added catalyst is disclosed in US 5227530 and SU 891145. Ru-added catalyst is disclosed in SU 978909.

The catalyst disclosed in US 5227530 contains Cu, Al, Cr and B as the essential components. This catalyst is prepared as follows. A catalyst precursor, comprising the foregoing four components, is subjected to a high temperature heat treatment at 600 °C to 1500 °C, thereby to prepare a structure which shows a unique X-ray diffraction. Subsequently, 0.05 to 50% of Pd or K may optionally be added thereto. The catalyst disclosed in SU 891145 is prepared by adding an alkali sodium hypophosphite solution to a mixed aqueous solution containing chlorides of Cu, Co and Pd to form a precipitate of phosphates, wherein Cu and Co in the final product of catalyst are present in the form of phosphates.

Further, the catalyst disclosed in SU 978909 contains Cu and Zn as main components and is added with Ru and BaO as promoters. An amount of these mixtures is 0.3 to 10 wt-%, and a mixing ratio of BaO to Ru is 2 : 1 (weight ratio), so that the catalyst contains Ru as the noble metal in the range of 0.1 to 3.3%.

In case that cyclohexanone is industrially produced by dehydrogenation of cyclohexanol, the reaction is usually carried out with keeping a conversion rate of cyclohexanol at a practically available level. Accordingly, in response to decrease of the catalyst activity, it is necessary to increase the reaction temperature in order to maintain the level of the conversion rate. As a result, there is no further choice to that a metal oxide based catalyst with a low thermal stability must be employed at an undesired temperature, thereby affecting the life-time of the catalyst and making it difficult to keep the stability in use of the catalyst.

The above-described operations for increasing the reaction temperature are essential and inevitable so far as the catalyst activity varies with time. However, if any highly active catalyst showing a much higher conversion rate than the practically available level could be developed, then a temperature of starting the operations may be set low, allowing for usage of the catalyst under milder temperature conditions and avoiding reduction of the performance due to the effect of heat load. In addition, the use of the highly active catalyst takes a long time period to achieve the necessary temperature increase, because of a large conversion rate decreasing range acceptable to the deterioration of activity with time which necessarily occurs in use. As a result, the highly active catalyst allows the stable use for a long time period and the extended catalyst life-time. Accordingly, a development of a highly active catalyst was desired.

### Summary of The Invention

The inventors have diligently investigated and found that addition of noble metals by a small amount to copper oxide as a main component results in a significant improvement in performance of the catalyst, especially excellency thereof may be obtained by addition of Pd, Pt and Ru. Further studies in details have completed the present invention.

By the way, only high activity is not enough to practicable properties needed for the industrial catalyst. But a high selectivity for increasing the yield of the intended product, or a sufficiently high mechanical strength for enduring a long time use without pulverization by handling the catalyst are also needed therefor. Those properties are not realizable by combining copper oxide with the noble metals only, and such catalyst is industrially unavailable. Therefore, the inventors have further investigated on further addition of each of a variety of additive components in order to provide the practically available properties and improve the performance of the catalyst.

As a result of the investigation, the invention for a catalyst for dehydrogenation of cyclohexanol was made. The catalyst is a copper oxide based catalyst including copper oxide and a noble metal as essential components, wherein the catalyst contains 20 to 400 ppm of at least any one element selected from Pd, Pt and Ru as the noble metal component, and also at least any one oxide of a metal selected from Al, Zn and Si as an additive component for substantially improving the practical catalytic properties as well as optionally contains at least any one selected from Na and Ca as an optional additive component for further improving the practicability. It should be noted that the additive component may be added alone, while the optional additive component should be added in combination with the additive component.

The method of preparation of the above is characterized in that a precipitating mother liquor is utilized, which contains a water-soluble compound or an oxide-sol of a copper component as a catalytic component at least one metal selected from Al, Zn and Si as an additive component, so as to form a precipitate through a neutralization reaction with an alkali and then wash it with water, before a hydroxide of at least one component selected from Na and Ca or a water-soluble compound thereof is further added as the optional additive component, followed by sequential processes of filtration, heat treatment and forming, thereby to provide an intermediate formed product. Alternately, the precipitating mother liquor of the copper component and the additive component is subjected to a neutralizing reaction to form a precipitate, followed by sequential processes for washing with water, filtration, heat treatment and forming, thereby to obtain a formed product before the formed product is further supported with a solution sprayed thereon, wherein the solution includes the water-soluble compound selected from nitrates, sulfates, chlorides and carbonates of at least one component of Na and Ca, thereby to obtain an intermediate formed product, and then the resulting intermediate formed product is further supported with an aqueous solution containing salts of the noble metal component, which is sprayed thereon.

A further method of preparation of the foregoing catalyst is characterized in that at least one copper component selected from copper oxide, copper hydroxide and basic copper carbonate is utilized as the catalytic component, while a compound of at least one metal selected from Al, Zn and Si is utilized as the additive component, and optionally, a further compound of at least one component selected from Na and Ca is used as the optional addition component. Those are subjected to either sequential processes of kneading, thermal treatment, and forming or other sequential processes of kneading, forming, and thermal treatment, thereby to obtain an intermediate formed product and then the resulting intermediate formed product is supported by an aqueous solution of salts of the noble metal component, which is sprayed thereon.

## Best Modes For Practice Of The Invention

In case of converting each component into its oxide in percentage, the catalyst of the present invention includes 20% - 97% of copper oxide as an essential component, and 20ppm - 400ppm (0.002% to 0.04%) of the noble metal, 3% - 80% of an additive component added to the catalyst for providing the practical properties, and 0.1% - 5% of an optional additive component optionally added to the catalyst in combination with the additive component.

If the content of the copper oxide is lower than 20%, then the catalytic activity is not enough. If the content of the copper oxide is higher than 97%, then a mechanical strength or a thermal resistance or a thermal stability is reduced to cause a practical problem. If the content of the noble metal is less than 20 ppm, then an activity-improving-effect is insufficient. If the content of the noble metal is more than 400 ppm, then the adding effect is saturated, so that a further increase in the amount of the additive causes not only a decrease in the activity-improving-effect but also another decrease in selectivity, which is a significantly negative effect. If the contents of the additive component and the optional additive component are out a range of 3 to 80% and another range of 0.1 to 5% respectively, then the catalyst becomes problematical for use in terms of the properties as the industrial catalyst, especially any of the mechanical strength, the thermal resistance or stability and the selectivity.

Further, the catalyst of the present invention may be prepared by a combination of a co-precipitation process with a spray process, wherein the copper component and the additive component are reacted with alkali in the co-presence of a water-soluble compound or an oxide-sol, thereby to obtain a precipitate, and further either a hydroxide or a water-soluble compound of an optional additive component is added thereto, before a noble metal component is sprayed thereon. Alternatively, the catalyst may also be prepared by another combination of a kneading process with a spray process, wherein all of the compounds except for the noble metal are subjected to the kneading process, the heat treatment and the forming process or alternatively subjected to the kneading process, the forming process and the heat treatment, thereby to obtain an intermediate product as formed, and subsequently the noble metal component is sprayed thereon. Source materials adjusted with each of the methods are selected.

In case that the catalyst is prepared through the co-precipitation operation, any water-soluble copper components are available. Notwithstanding, sulfate, nitrate and chloride and the like are economically preferred. In other case that the catalyst is prepared through the kneading operation, it is necessary that the copper compound is free of any catalytic poison. Practically, basic copper carbonate, copper hydroxide, and copper oxide and the like are preferred.

Since the noble metal component is added by the spray process, it is necessary that the component is water-soluble. Palladium chloride, sodium palladium chloride, palladium nitrate, palladium sulfate, tetrachloro palladium salts, dichloroaminepalladium, and dinitro-polyaminepalladium are preferred for the palladium ingredient. Platinum chloride, platinum nitrate, and dinitrodiamineplatinum and the like are preferred for the platinum ingredient. Ruthenium chloride and ruthenium nitrate are preferred for the ruthenium ingredient.

In case that the catalyst is prepared through the co-precipitation operation, the water-soluble compound or the oxide-sol is used as a source material for the additive component. It is practically preferable that this water-soluble compound is selected from nitrate, sulfate and chloride of at least one metal selected from Al, Zn and Si. The hydroxide or the water-soluble compound may be used as a source material for the optional additive component. It is practically preferable that these compounds is hydroxide, carbonate, nitrate, sulfate or chloride of at least one component selected from Na and Ca. A precipitate of the copper component and the additive component is formed, followed by washing and addition onto a slurry of the precipitate, or by spraying the same before the spray of the noble metal component or by spraying the same together with the noble metal component.

In case that the catalyst is prepared by the kneading operation, it is preferable that source materials of the additive component and the optional additive component are compounds free of any catalytic poisons. It is practically preferable that the additive component is carbonate, hydroxide or oxide of at least one metal selected from Al, Zn and Si. It is also preferable that the optional additive component is carbonate, hydroxide or oxide of at least one element selected from Na and Ca.

In case that the catalyst is prepared through the co-precipitation operation, a precipitating mother liquor containing a dissolved water-soluble compound of the copper component and the additive component is prepared. If the oxide-sol is used as the additive component, another precipitating mother liquor containing a suspended oxide-sol is prepared. A precipitate is then formed by a neutralization reaction with alkali, followed by washing, addition of an optional additive component, drying and calcination processes, then extrude or tablet to obtain an intermediate product as formed. Alternatively, the precipitate is washed with water, dried, calcined and formed before an aqueous solution containing the optional additive component is sprayed thereto prior to the calcination process, thereby to obtain an intermediate product as formed. Further, a solution of the noble metal component is then sprayed on the obtained intermediate product as formed, followed by the calcination process to obtain a final product of the catalyst.

In case that the catalyst is prepared through the kneading operation, a water is added to compounds as source materials other than the noble metal component for kneading the same, followed by drying, and calcination processes prior to the forming process. In case of forming into extrusions, after the kneading process, the forming process may be taken place, followed by the drying and calcination processes. An aqueous solution of the noble metal compound, which has already been prepared, is sprayed onto the obtained intermediate product as formed prior to the calcination process to obtain a final product of the catalyst.

A heat treatment in the process for preparing the catalyst is carried out in order to provide a mechanical stability to the formed catalyst. A calcination temperature of the heat treatment is preferably in the range of 300 °C to 500 °C. If the calcination temperature is less than 300 °C, then the mechanical strength in use for the reaction is not sufficient. If the calcination temperature is higher than 500 °C, then a crystal growth of the copper oxide is caused, thereby making it difficult to obtain a catalyst exhibiting high performance. Accordingly, the catalyst of the present invention is significantly different from the noble-metal-containing-catalyst calcined at 600 °C to 1500 °C, which is disclosed in US Patent 5,227,530. Calcination at such high temperature causes the catalyst of the present invention to lose its superior activity.

It is important for the catalysts of the present invention that the content of the noble metal component to be added to the catalyst is 20 to 400 ppm (0.002 to 0.04 %). If the content of the noble metal is 0.05 to 50% or 0.1 to 3.3% as disclosed in US Patent 5227530 and SU Patent 978909, then the catalytic selectivity is reduced so as not available practically. Accordingly, the catalyst of the present invention is substantially different from the known catalysts, and thus is not readily presumable from the foregoing knowledge.

Still another noble metal based catalyst is disclosed in SU Patent 891145. Since a mixed solution of alkali hypophosphite with potassium hydroxide is used as a precipitating agent, the catalytic component is precipitated and present in the form of phosphates in the catalyst. This known catalyst is different from the catalyst of the present invention, wherein the catalytic component is present in the form of oxide. Further, the content of the noble metal in this known catalyst is high, for example, 0.1 to 3.3%, in the light of which the known catalyst is significantly different from the catalyst of the present invention.

**Examples**

The present invention will be described in detail with reference to examples. Performance of the catalyst of the present invention was confirmed by reduction of the catalyst with hydrogen and subsequent dehydrogenation reaction of cyclohexanol. Conditions for catalyst reduction and examination, and methods for calculations of activity and selectivity as the catalyst performance are as follows:

**1. Conditions for the catalyst reduction and tests**

| Amount of the catalyst | 5 ml |
|---|---|

Conditions for reduction

| Gas flow rate | 1.6 L/min |
|---|---|
| Temperature | 200 °C |
| Time | 17 hr. |
| Gas composition | hydrogen 2 % and nitrogen 98 % |
| Amount of the catalyst | 5 ml |

Conditions for the test

| LHSV | 5 hr.$^{-1}$ |
|---|---|
| Pressure | normal pressure |
| Reaction temperatures | 220 °C, 240 °C and 260 °C |
| Reaction time | 30 hr. |
| Composition of source materials | |
| Cyclohexanol | 98 % |
| Water | 2 % |

**2. Method of calculation of the activity and the selectivity**

The activity and the selectivity as the catalyst performance were calculated as follows:
Activity: Respective contents of cyclohexanol in the reactants and in the products at each of the reaction temperatures were determined by a gas chromatography. Conversion rates were obtained based on the following equation, and the obtained conversion rates were then averaged to determine the catalyst performance:

$$\text{Conversion Rate (\%)} = (A - B) / (A) \times 100$$

**Selectivity:** Respective contents of cyclohexanol in the reactant and the product as well as a content of cyclohexanone in the product were determined by the gas chromatography. The selectivity was determined according to the following equation to show a relationship between the selectivity and the conversion rate in the drawing. A selectivity rate at the cyclohexanol conversion rate of 50% was found by interpolation, thereby to define the selectivity.

$$\text{Selectivity (\%)} = (C) / (A - B) \times 100$$

wherein A: concentration of cyclohexanol in the reactant (%)
B: concentration of cyclohexanol in the product (%)
C: concentration of cyclohexanone in the product (%)

**Example-1**

0.95 kg of copper sulfate and 2.31 kg of zinc sulfate were weighed and 10 L of a pure water was added with agitation/dissolution to form a solution A. Separately, 1.27 kg of sodium carbonate was weighed and 10 L of the pure water was added and dissolved to prepare a solution B. The solution B was slowly dropped for 100 minutes into the solution A, which had already been intensively agitated, thereby to form a precipitate. A slurry of the precipitate was filtered and calcined in the air at 350 °C for 2 hours. The calcined product was then washed with water and dried at 110 °C for 20 hours. Subsequently, the dried product was granulated and formed into tablets.

100g of the obtained tablets were transferred into a beaker. The beaker was placed on a rotary spraying device in order to rotate the beaker, so that 10 ml of an aqueous solution containing 0.1 % of palladium nitrate was sprayed onto the tablets. After the spraying, a calcination process was taken place in the air at 350 °C for 2 hours, thereby to obtain a catalyst of Example-1. Components and compositions of this catalyst and the performance test results are as shown in Table-1 and Table-2, respectively.

**Example-2**

A catalyst of Example-2 was prepared in the same processes as Example-1, except that the amounts of copper sulfate and zinc sulfate were 1.58 kg and 1.60 kg respectively. Components and compositions of this catalyst and the performance test results are as shown in Table-1 and Table-2, respectively.

**Example-3**

A catalyst of Example-3 was prepared in the same processes as Example-1, except that the amounts of copper sulfate and zinc sulfate were 2.53 kg and 0.53 kg respectively. Components and compositions of this catalyst and the performance test results are as shown in Table-1 and Table-2, respectively.

**Comparative Example-1**

A catalyst of Comparative Example-1 was prepared in the same process as Example-1, except that the process for spraying 10 ml of the aqueous solution containing 0.1% of palladium nitrate onto the tablets and the subsequent thermal treatment at 350 °C were not carried out. Components and compositions of this catalyst and the performance test results are as shown in Table-1 and Table-2, respectively.

**Example-4**

0.95 kg of copper sulfate, 2.31 kg of zinc sulfate and 0.31 kg of aluminum sulfate were weighed and added with 10 L of a pure water for agitation thereof, thereby to obtain a dissolved solution A. Separately from this solution, 1.58 kg of sodium carbonate was weighed and added with 10 L of another pure water for agitation thereof, thereby to obtain another dissolved solution B. The solution B was gradually dropped into the solution A as intensively agitated, thereby to form a precipitate. A precipitated slurry was filtered and calcined in the air at 350 °C for 2 hours, followed by washing the calcined product with water and subsequently drying the same at 110 °C for 20 hours. The dried product was then granulated and made into tablets.

100g of the obtained tablets was transferred into a beaker, which is then placed on a rotary spraying device, so as to rotate the beaker and to spray 6 ml of an aqueous solution containing 4.8% of sodium carbonate onto the tablets, followed by calcining the same in the air at 150 °C for 20 hours. Further, 100 g of these tablets was transferred again to a beaker, which is then placed on the rotary spraying device in order to rotate the beaker. 10 ml of an aqueous solution containing 0.1% of palladium nitrate was sprayed onto the tablets. After spraying, the product was calcined in the air at 350 °C for 2 hours, thereby to obtain a catalyst of Example-4. Components and compositions of this catalyst and the performance test results are as shown in Table-1 and Table-2, respectively.

**Example-5**

The same processes were taken place as in Example-4 to prepare a catalyst of Example-5, except that 10 ml of an aqueous solution containing 0.2% of palladium nitrate was sprayed, instead of spraying 10 ml of the aqueous solution containing 0.1% of palladium nitrate in Example-4. Components and compositions of this catalyst and the performance test results are as shown in Table-1 and Table-2, respectively.

**Example-6**

The same processes were taken place as in Example-4 to prepare a catalyst of Example-6, except that 10 ml of an aqueous solution containing 0.4% of palladium nitrate was sprayed, instead of spraying 10 ml of the aqueous solution containing 0.1% of palladium nitrate in Example-4. Components and compositions of this catalyst and the performance test results are as shown in Table-1 and Table-2, respectively.

**Comparative Example-2**

The same processes were taken place as in Example-4 to prepare a catalyst of Comparative Example-2, except that the processes for spraying 10 ml of the aqueous solution containing 0.1% of palladium nitrate onto tablets and subsequent calcination at 350 °C in Example-4 were not carried out. Components and compositions of this catalyst and the performance test results are as shown in Table-1 and Table-2, respectively.

**Comparative Example-3**

The same processes were taken place as in Example-4 to prepare a catalyst of Comparative Example-3, except that 10 ml of an aqueous solution containing 0.5% of palladium nitrate was sprayed, instead of spraying 10 ml of the aqueous solution containing 0.1% of palladium nitrate in Example-4. Components and compositions of this catalyst and the performance test results are as shown in Table-1 and Table-2, respectively.

**Example-7**

0.98 kg of basic copper carbonate, 0.28 kg of silica-sol and 0.035 kg of calcium carbonate were weighed and added with 200 ml of a pure water and further agitated well, followed by calcining the same at 600 °C for 3 hours and making the same into tablets. Further, 100 g of these tablets were put into a beaker, which is placed on the rotary spraying device to rotate the beaker, and the tablets were sprayed with 10 ml of an aqueous solution containing 0.1% of palladium nitrate. After spraying, the tablets were calcined in the air at 350 °C for 4 hours, thereby to prepare a catalyst of Example-7. Components and compositions of this catalyst and the performance test results are as shown in Table-1 and Table-2, respectively.

**Comparative Example-4**

The same processes were taken place as in Example-7 to prepare a catalyst of Comparative Example-4, except that the processes for spraying 10 ml of the aqueous solution containing 0.1% of palladium nitrate onto the tablets and subsequent calcination at 350 °C in Example-7 were not carried out. Components and compositions of this catalyst and the performance test results are as shown in Table-1 and Table-2, respectively.

**Example-8**

2.20 kg of copper sulfate, 0.65 kg of zinc sulfate, 0.26 kg of aluminum sulfate and 0.1 kg of a 20%-silica-colloid solution were weighed, and added with 10 L of a pure water, and further agitated for dissolution thereof so as to obtain a solution A, wherein silica-colloids are dispersed in the aqueous solution of salts. Separately from this solution, 1.58 kg of sodium carbonate was weighed and added with 10 L of another pure water for dissolution thereof, thereby to obtain a solution B. The solution B was gradually dropped with taking 100 minutes into the solution A as intensively agitated, thereby to obtain a precipitate. The precipitate slurry was filtered and calcined in the air at 350 °C for 2 hours, followed by washing the calcined product with water and drying the same at 110 °C for 20 hours. The dried product was then granulated and formed into tablets.
100 g of the obtained tablets were transferred into a beaker which is then placed onto a rotary spraying device so as to rotate the beaker. 10 ml of an aqueous solution containing 0.1% of palladium nitrate was sprayed onto the tablets. After spraying, the tablets were calcined in the air at 350 °C for 2 hours, to obtain a catalyst of Example-8. Components and compositions of this catalyst and the performance test results are as shown in Table-1 and Table-2, respectively.

**Example-9**

The same processes were taken place as in Example-8 to prepare a catalyst of Example-9, except that 10 ml of an aqueous solution containing 0.1 % of platinum nitrate was sprayed, instead of spraying 10 ml of the aqueous solution containing 0.1% of palladium nitrate in Example-8. Components and compositions of this catalyst and the performance test results are as shown in Table-1 and Table-2, respectively.

**Comparative Example-5**

The same processes were taken place as in Example-8 to prepare a catalyst of Comparative Example-5, except that the processes for spraying 10 ml of the aqueous solution containing 0.1% of palladium nitrate onto the tablets and subsequent calcination at 350 °C in Example-8 were not carried out. Components and compositions of this catalyst and the performance test results are as shown in Table-1 and Table-2, respectively.

**Example-10**

1.01 kg of copper sulfate was weighed and added with 3.7 L of a pure water and dissolved to prepare a solution A. Separately from this solution, 0.410 kg of sodium carbonate and 0.244 kg of a water-glass containing 29% of silicon oxide were weighed and added with 10 L of a pure water, followed by agitation and dissolution thereof, thereby to prepare a solution B. The solution A was gradually dropped with taking 120 minutes into the solution B as intensively agitated, thereby to obtain a precipitate followed by a continuous additional agitation thereof for 60 minutes, thereby to age the precipitate. The precipitate was washed with water and then added with 0.014 kg of calcium hydroxide, and further filtered and calcined in the air at 350 °C for 2 hours. The resulting calcined product was granulated and formed into tablets.

100 g of the obtained tablets were transferred into a beaker which is then placed on a rotary spraying device, in order to rotate the beaker. 10 ml of an aqueous solution containing 0.1% of palladium nitrate was sprayed onto the tablets. After spraying, the product was calcined in the air at 350 °C for 2 hours, thereby to obtain a catalyst of Example-10. Components and compositions of this catalyst and the performance test results are as shown in Table-1 and Table-2, respectively.

**Example-11**

The same processes were taken place as in Example-10 to prepare a catalyst of Example-11, except that 10 ml of an aqueous solution containing 0.1% of ruthenium nitrate was sprayed, instead of spraying 10 ml of the aqueous solution containing 0.1% of palladium nitrate in Example-10. Components and compositions of this catalyst and the performance test results are as shown in Table-1 and Table-2, respectively.

**Example-12**

The same processes were taken place as in Example-10 to prepare a catalyst of Example-12, except that 10 ml of an aqueous solution containing 0.05% of ruthenium nitrate was sprayed, instead of spraying 10 ml of the aqueous solution containing 0.1% of palladium nitrate in Example-10. Components and compositions of this catalyst and the performance test results are as shown in Table-1 and Table-2, respectively.

**Comparative Example-6**

The same processes were taken place as in Example-10 to prepare a catalyst of Comparative Example-6, except that the processes for spraying 10 ml of the aqueous solution containing 0.1% of palladium nitrate onto the tablets and subsequent calcination at 350 °C in Example-10 were not carried out. Components and compositions of this catalyst and the performance test results are as shown in Table-1 and Table-2, respectively.

**Example-13**

2.20 kg of copper sulfate was weighed and added with 7.4 L of a pure water, followed by agitation and dissolution thereof, thereby to form a solution A. Separately from this solution, 0.466 kg of sodium carbonate and 0.127 kg of a water-glass containing 29% of silicon oxide were weighed and added with 10 L of another pure water for agitation and dissolution thereof, thereby to prepare a solution B. The solution A was gradually dropped with taking 120 minutes into the solution B as intensively agitated, thereby to produce a precipitate. Further, an additional continuous agitation thereof was taken place for 60 minutes, thereby to age the precipitate. The precipitate was washed with water, followed by filtering the precipitate slurry and calcining the same in the air at 350 °C for 2 hours. The resulting calcined product was granulated and formed into tablets.

100g of the obtained tablets were transferred into a beaker which is then placed on a rotary spraying device for rotating the beaker, and 5 ml of an aqueous solution containing 0.1% of ruthenium nitrate was sprayed onto the tablets. After spraying, the product was calcined in the air at 350 °C for 2 hours, thereby to obtain a catalyst of Example-13. Components and compositions of this catalyst and the performance test results are as shown in Table-1 and Table-2, respectively.

**Example-14**

The same processes were taken place as in Example-13 to prepare a catalyst of Comparative Example-14, except that 0.076 kg of the water-glass containing 29% of silicon oxide was added, instead of 0.127 kg thereof in Example-13, thereby to prepare a precipitate followed by washing the same with water, and then 0.025 kg of calcium hydroxide was further added thereto, and a precipitate slurry was filtered, calcined and formed and further added with 3 ml of an aqueous solution containing 0.1% of ruthenium nitrate, instead of 5 ml of the aqueous solution containing 0.1% of ruthenium nitrate. Components and compositions of this catalyst and the performance test results are as shown in Table-1 and Table-2, respectively.

**Comparative Example-7**

The same processes were taken place as in Example-13 to prepare a catalyst of Comparative Example-7, except that the processes for spraying 10 ml of the aqueous solution containing 0.1% of ruthenium nitrate onto the tablets and subsequent calcination at 350 °C in Example-13 were not carried out. Components and compositions of this catalyst and the performance test results are as shown in Table-1 and Table-2, respectively.

Table-1

| | CuO (%) | noble metal (ppm) | | | Additive component (%) | | | optional addition component (%) | |
|---|---|---|---|---|---|---|---|---|---|
| Components and Compositions of Catalysts | | | | | | | | | |
| | | Pd | Pt | Ru | ZnO | $Al_2O_3$ | $SiO_2$ | $Na_2O$ | CaO |
| Ex.-1 | 32 | 100 | | | 68 | | | | |
| comp.-1 | 32 | 0 | | | 68 | | | | |
| Ex.-2 | 53 | 100 | | | 47 | | | | |
| Ex.-3 | 84 | 100 | | | 16 | | | | |
| Ex.-4 | 30 | 100 | | | 65 | 4.7 | | 0.3 | |
| Ex.-5 | 30 | 200 | | | 65 | 4.7 | | 0.3 | |
| Ex.-6 | 30 | 400 | | | 65 | 4.7 | | 0.3 | |
| comp.-2 | 30 | 0 | | | 65 | 4.7 | | 0.3 | |
| comp.-3 | 30 | 1000 | | | 65 | 4.7 | | 0.3 | |
| Ex.-7 | 71 | 100 | | | | | 29 | | |
| comp.-4 | 71 | 0 | | | | | 29 | | |
| Ex.-8 | 75 | 100 | 0 | | 19 | 4.0 | 2.0 | | |
| Ex.-9 | 75 | 0 | 100 | | 19 | 4.0 | 2.0 | | |
| comp.-5 | 75 | 0 | 0 | | 19 | 4.0 | 2.0 | | |
| Ex.-10 | 80 | 100 | | 0 | | | 18 | | 2.0 |
| Ex.-11 | 80 | 0 | | 100 | | | 18 | | 2.0 |
| Ex.-12 | 80 | 0 | | 50 | | | 18 | | 2.0 |
| comp.-6 | 80 | 0 | | 0 | | | 18 | | 2.0 |
| Ex.-13 | 95 | | | 50 | | | 5.0 | | 0 |
| Ex.-14 | 95 | | | 30 | | | 3.0 | | 2.0 |
| comp.-7 | 95 | | | 0 | | | 5.0 | | 0 |

Table-2

| Catalyst Performance for Dehydrogenation of Cyclohexanol | | |
|---|---|---|
| | Test Results of Performance | |
| | Conversion Rate (%) | Selectivity (%) |
| Ex.-1 | 55.2 | 99.5 |

Table-2 (continued)

| Catalyst Performance for Dehydrogenation of Cyclohexanol | | |
|---|---|---|
| | Test Results of Performance | |
| | Conversion Rate (%) | Selectivity (%) |
| comp.-1 | 49.5 | 98.2 |
| Ex.-2 | 57.6 | 99.4 |
| Ex.-3 | 59.7 | 99.6 |
| Ex.-4 | 54.3 | 99.5 |
| Ex.-5 | 56.0 | 99.6 |
| Ex.-6 | 57.8 | 99.3 |
| comp.-2 | 48.4 | 98.3 |
| comp.-3 | 57.1 | 96.8 |
| Ex.-7 | 56.9 | 99.8 |
| comp.-4 | 50.0 | 98.8 |
| Ex.-8 | 59.5 | 99.1 |
| Ex.-9 | 58.2 | 99.4 |
| comp.-5 | 51.7 | 98.7 |
| Ex.-10 | 59.8 | 99.6 |
| Ex.-11 | 58.3 | 99.8 |
| Ex.-12 | 55.0 | 99.7 |
| comp.-6 | 52.4 | 98.6 |
| Ex.-13 | 58.8 | 99.7 |
| Ex.-14 | 57.1 | 99.8 |
| comp.-7 | 53.9 | 99.2 |
| Note) Conversion Rate: Averaged value of respective conversion rates at respective reaction temperatures (220 °C, 240 °C and 260 °C) Selectivity: Selectivity at 50% conversion rate | | |

**Industrial Applicability**

As described above, it was confirmed that the catalyst of the present invention has an extremely high activity and an extremely high selectivity for allowing a production of cyclohexanone at a high yield, and the performance of this catalyst is remarkably excellent as compared to the conventional catalysts.

The catalyst of the present invention has very high performance, and ensures, in the practical use, the more practical conversion rate of cyclohexanol at a lower reaction temperature than that of the conventional catalysts. Besides, the catalyst of the present invention allows for use under the lower thermal-load condition than that of the conventional catalysts, and for improvement in the practical properties of the catalyst, thereby to obtain the stability of the performance for a long time period.

**Claims**

1.   A catalyst for dehydrogenation of cyclohexanol, said catalyst being a copper oxide based catalyst which includes essential components of copper oxide and a noble metal, wherein said catalyst contains 20 to 400 ppm of at least any one element selected from Pd, Pt and Ru as a component of said noble metal, and an oxide of at least one metal selected from Al, Zn and Si as an additive component.

2.   The catalyst for dehydrogenation of cyclohexanol as claimed in claim 1, further containing at least one of Na and Ca as an optional additive component.

3.   The catalyst for dehydrogenation of cyclohexanol as claimed in claim 1 or 2, wherein, based on conversion of catalyst component into oxide, a content of Cu is 20 to 97%, a content of said additive component is 3 to 80%, and a content of the optional additive component is 0.1 to 5%.

4. A method of preparing a catalyst for dehydrogenation of cyclohexanol as claimed in any one of claims 1 to 3, wherein said method comprises the steps of : preparing a precipitating mother liquor which contains a copper component and either a water-soluble compound or an oxide-sol of at least one metal selected from Al, Zn and Si as an additive component; producing a precipitate through a neutralization reaction with alkali ; subjecting the precipitate with washing, heat treatment, and forming, thereby to obtain an intermediate product; and subsequently spraying a solution, which contains salts of said noble metal component, onto said intermediate product as obtained, so that said solution as sprayed is supported on said intermediate product.

5. The method of preparing the catalyst for dehydrogenation of cyclohexanol as claimed in claim 4, wherein a compound of at least one component selected from Na and Ca is further added as said optional additive component to a slurry which has been prepared by having produced said precipitation from said copper component and said additive component and having completed said washing process.

6. The method of preparing the catalyst for dehydrogenation of cyclohexanol as claimed in claim 4, wherein a solution containing a water-soluble compound of at least one component selected from Na and Ca as an optional additive component is further sprayed and supported on said intermediate product.

7. A method of preparing the catalyst for dehydrogenation of cyclohexanol as claimed in any one of claims 1 to 3, wherein at least one component selected from copper oxide, copper hydroxide and basic copper carbonate is used as a copper component, and a compound of at least one metal selected from Al, Zn and Si is used as an additive component, and they are subjected to either sequential processes of kneading, heat treatment and forming or other sequential processes of kneading, forming and heat treatment, thereby to obtain an intermediate product as formed and subsequently an aqueous solution containing salts of a noble metal component is sprayed and supported onto said intermediate product as formed.

8. The method of preparing the catalyst for dehydrogenation of cyclohexanol as claimed in claim 7, wherein a compound of at least one component selected from Na and Ca is further added as an optional additive component during said kneading process.

# INTERNATIONAL SEARCH REPORT

| | |
|---|---|
| International application No. | |
| | PCT/JP02/03223 |

**A. CLASSIFICATION OF SUBJECT MATTER**
Int.Cl$^7$ B01J23/89, C07C45/29, 49/403

According to International Patent Classification (IPC) or to both national classification and IPC

**B. FIELDS SEARCHED**

Minimum documentation searched (classification system followed by classification symbols)
Int.Cl$^7$ B01J21/00-37/36, C07C45/29, 49/403

Documentation searched other than minimum documentation to the extent that such documents are included in the fields searched

| | | | |
|---|---|---|---|
| Jitsuyo Shinan Koho | 1926-1996 | Toroku Jitsuyo Shinan Koho | 1994-2002 |
| Kokai Jitsuyo Shinan Koho | 1971-2002 | Jitsuyo Shinan Toroku Koho | 1996-2002 |

Electronic data base consulted during the international search (name of data base and, where practicable, search terms used)
WPI/L, JOIS

**C. DOCUMENTS CONSIDERED TO BE RELEVANT**

| Category* | Citation of document, with indication, where appropriate, of the relevant passages | Relevant to claim No. |
|---|---|---|
| A | CN 1235870 A (Univ. Nankai),<br>14 November, 1999 (14.11.99),<br>(Family: none) | 1-8 |
| A | RU 978909 A (AS Beko Phys. Organ. Chem.),<br>07 December, 1982 (07.12.82),<br>(Family: none) | 1-8 |
| A | US 5227530 A (Amoco Corp.),<br>13 July, 1993 (13.07.93),<br>(Family: none) | 1-8 |
| A | WO 97008119 A (Korea Research Institute of Chemical Technology),<br>06 March, 1997 (06.03.97),<br>& EP 857169 A1 & US 6075170 A<br>& KR 169189 B & JP 11-509859 A | 1-8 |

| | | | |
|---|---|---|---|
| ☒ | Further documents are listed in the continuation of Box C. | ☐ | See patent family annex. |

| | |
|---|---|
| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br>24 June, 2002 (24.06.02) | Date of mailing of the international search report<br>09 July, 2002 (09.07.02) |
| Name and mailing address of the ISA/<br>Japanese Patent Office | Authorized officer |
| Facsimile No. | Telephone No. |

Form PCT/ISA/210 (second sheet) (July 1998)

EP 1 382 387 A1

**INTERNATIONAL SEARCH REPORT**

International application No.

PCT/JP02/03223

C (Continuation). DOCUMENTS CONSIDERED TO BE RELEVANT

| Category* | Citation of document, with indication, where appropriate, of the relevant passages | Relevant to claim No. |
|---|---|---|
| A | EP 204046 A2 (Industrial Technology Research Institute), 10 December, 1986 (10.12.86), & US 4670605 A & BR 8602499 A & JP 61-282334 A | 1-8 |

Form PCT/ISA/210 (continuation of second sheet) (July 1998)

13